# EUROPÄISCHE PATENTANMELDUNG

(11) **EP 4 368 695 A1**
(43) Veröffentlichungstag der Anmeldung: **15.05.2024**
(21) Anmeldenummer: 22206734.0
(22) Anmeldetag: 10.11.2022
(51) Int. Cl.: C12M 1/00, C12M 1/33

(54) **VERFAHREN UND VORRICHTUNG ZUR AUFBEREITUNG VON FLÜSSIGEN ORGANISCHEN SUBSTRATEN IN ANAEROBEN ANLAGEN UND PROZESSEN MITTELS VERKNÜPFUNG VON MECHANISCHER, VACUUM- SOWIE MEHRSTUFIGER ULTRASCHALL- UND PLASMA BEHANDLUNG**

(71) Anmelder: PRE Power Recycling Energyservice GmbH, 17033 Neubrandenburg Mecklenburg-Vorpommern (DE); Biogastechnik Süd GmbH, 88316 Isny Baden-Württemberg (DE)
(72) Erfinder: MAIER, Clemens, 88316 Isny (DE); MAIER, Gregor, 88316 Isny (DE); ROSSOW, Norbert, 17217 Penzlin (DE)
(74) Vertreter: Wehlan, Helmut

(57) **Zusammenfassung**

Die Erfindung betrifft ein Verfahren und eine Vorrichtung zur Aufbereitung von flüssigen organischen Substraten in anaeroben Anlagen und Prozessen mittels Verknüpfung von mechanischer, Vacuum- sowie mehrstufiger Ultraschall- und Plasma Behandlung. Das Verfahren durch folgende Schritte gekennzeichnet: Mechanische Zerkleinerung, Separationsstufe durch Behandlung mit Unterdruck und Überdruck, Ultraschall Behandlung oder/und Kombinierte Ultraschall-Plasma-behandlung, Ultraschallbehandlung eines zweiten Stromes aus Druckbehandlung, wobei diese Behandlungsstufen hintereinander geschaltet sind.

Die Vorrichtung zur Durchführung des Verfahrens umfasst einen Rotations-Schneider, ein Separationssystem aus dem nur flüssige Fraktionen mit unterschiedlichem Eindickungsgrad austreten, Geräte zur Druck- bzw. Unterdruckerzeugung, Geräte zur Plasmabehandlung, Geräte zur Ultraschallbehandlung, Geräte zur kombinierten Plasma und Ultraschallbehandlung in gemeinsamen Reaktionskammern sowie Gasspeicher.

Gegenstand der Erfindung ist auch eine Reaktorkammer für die kombinierte gleichzeitige Suspensionsbehandlung.

## Beschreibung

Die Erfindung betrifft eine Vorrichtung und ein Verfahren mit dem Ziel, die organischen Bestandteile einer inhomogenen Suspension maximal aufzuschließen und diese in einem vorrangig anaeroben Stoffwechselprozess nahezu vollständig zur Bildung von Methan und Wasserstoff umzusetzen. Die Vorrichtung zur Durchführung des Verfahrens umfasst einen Rotations-Schneider, ein Separationssystem aus dem nur flüssige Fraktionen mit unterschiedlichem Eindickungsgrad austreten, Geräte zur Druck- bzw. Unterdruckerzeugung, Geräte zur Plasmabehandlung, Geräte zur Ultraschallbehandlung, Geräte zur kombinierten Plasma und Ultraschallbehandlung in gemeinsamen Reaktionskammern sowie Gasspeicher.

Gegenstand der Erfindung ist auch eine Reaktorkammer für die kombinierte gleichzeitige Suspensionsbehandlung.

In dem Verfahren sind mehrere Behandlungsstufen zum maximalen Aufschluss der organischen Inhaltsstoffe der flüssigen Suspension in eine gelöste Form hintereinandergeschaltet. Die Flüssigkeit durchläuft je nach Beschaffenheit und Inhaltsstoffen diese Stufen nacheinander oder zyklisch oder einzeln in angepasster Reihenfolge.

Die Behandlungsstufen erfolgen in separaten Geräte- und Apparate-Konfigurationen als ein Teilprozess ab und erzielen einen vollständigen Aufschluss der organischen Inhaltsstoffe in die gelöste Form zur Umsetzung in biochemischen Reaktionen vorrangig in anaeroben wie teilweise auch in aeroben Abwasser- und Abfallaufbereitungsanlagen sowie Biogaserzeugungsanlagen.

Durch die kombinierte Ultraschall-Plasmabehandlung wird aus anorganischen Verbindungen mit Wasserstoffanteil - wie z.B. NH₃ - zusätzlicher Wasserstoff gebildet. Neben der Wasserstoffbildung werden auch geringe Mengen von Synthesegas CO und auch CO₂ gebildet. Diese Gase werden dem Fermentationsprozess zur Steigerung der Methanbildungseffizienz zugeführt.

In dem Verfahren tritt eine Entgasung des zu behandelnden Substrates ein, größter Gasbestandteil des entgasten Substrates ist CO₂ mit 60-80 Vol. %. Schwefelwasserstoff wird - je nach Ausgangssubstrat - ebenfalls ausgegast. Dieses Gas wird einer Plasmabehandlung durch einen Stack-Reaktor zugeführt und wird auf diesem Wege zu Methan, Methanol sowie Synthesegas umgewandelt. Diese durch die Plasmabehandlung entstandenen Gase werden dem Fermentationsprozess zur Stabilisierung und Effizienzsteigerung zugeführt sowie einer externen Nutzung über eine Gasreinigungsstufe und entsprechende Gaslager zugeführt.

Das Verfahren stellt eine neue gezielte Verknüpfung von Effekten aus bekannten und neuen Behandlungsverfahren von Gärsubstraten, Klärschlämmen sowie verflüssigten organischen Abfallfraktionen sowie stark organisch belastetem Wasser dar.

Zu behandelnde Fraktionen durchlaufen den Prozess, wobei entsprechend der Zusammensetzung und Qualität der Stoffe bestimmte Stufen nur anteilig oder eben auch zyklisch durchströmt werden.

Die entstehenden Gase werden teilweise dem anaeroben Fermentationsprozess zur Stabilisierung und Reaktionsverstärkung zugeführt, parallel ist eine externe Nutzung über Aufbereitungs-/ Reinigungsstufen und Zwischenlager beabsichtigt.

### Stand der Technik

Die Druckschrift WO2007/115660 A2 betrifft ein Verfahren und eine Vorrichtung zur Herstellung stickstoffhaltiger Düngemittel durch die Behandlung biogener Stoffe. Die beiden beim Biogasprozess entstehenden und in der Flüssigkeit gelösten Gase Ammoniak und Kohlendioxid werden aus der Flüssigkeit entfernt und einer Nutzung zugeführt. Dabei wird u.a. Ultraschall eingesetzt.

Die Offenlegungsschrift EP 2 243 823 A2 beschreibt ein Verfahren und eine Vorrichtung zur Erzeugung und Verteilung von Energie, bei dem vorzugsweise lignocellulosehaltige Rohstoffe zu einem flüssigen Gärsubstrat aufbereitet werden und dieses einem Biovergasungsprozess zugeführt wird.

Eine verbesserte Fermentervorrichtung zur Umwandlung von nassen kohlenstoffhaltigen Biomassematerialien in Biogas ist Gegenstand von US 2003 0 173 291 A1, umfassend eine Fermentationseinheit, Mittel zur Förderung von aufgeschlämmter wässriger Biomasse, Mittel zum Fördern konzentrierter wässriger Biomasse, Mittel zum Entfernen von Abfallfeststoffen aus der Druckschwingungskomponente und eine Druckschwingungspumpe zum Steuern des Drucks. Die Vorrichtung umfasst einen programmierbaren Computer, der mit einer Datenbank ausgestattet ist, die zuvor gemessene Biomasse-Biogas-Umwandlungsdaten für Biomassematerialien unterschiedlicher Zusammensetzung mit Werten für die erste und zweite Druckphase in Beziehung setzt. Der Computer überwacht kontinuierlich den Druck des Biogases in der Druckwechselkomponente und passt den Zyklus der Druckwechselpumpe an, um die Biogasumwandlung von Biomasse aus der Biomassequelle zu optimieren.

Die Offenlegungsschrift DE 10 2014 210 346 A1 bezieht sich auf ein Verfahren und die Vorrichtung einer gezielten Einbringung und Kultivierung einer Schwimmschicht zur Erhöhung und Steuerung der Besiedlungsdichte aktiver Bakterien und Archaeen unter Nutzung mehrstufiger selbstregulierender Ultraschall-Behandlung von Flüssigkeiten in Biogasfermentern. Indem in die jeweils energetisch umzusetzenden Gärsubstrate zusätzlich grobfaserige Materialien eingeleitet, hieraus separate Schichten für eine Ansiedlung und Konzentrierung von Bakterien und Archaeen geschaffen und durch die spezifische Anlagenkonfiguration - abhängig von der Fermenterorganik - optimiert werden, beschleunigt sich die Substratumsetzung, und die Schwimmschicht ersetzt physikalische Einrichtungen, welche die Steigerung der Raumbelastung ermöglichen sowie den Austrag von methanbildenden Substraten aus dem quasikontinuierlichen Fermenter weitestgehend mindern. Die Ultraschall-Behandlung dient der Optimierung des Systems in Hinblick auf Faseraufschluss und hydraulische Bedingungen.

Die Druckschrift WO2021/188301 A1 sieht die Abtrennung und Verbrennung von mindestens einem Kohlenwasserstoff, einer Sauerstoffverbindung, einer Schwefelverbindung und/oder einer Stickstoffverbindung aus Industriegas oder aus durch Vergasung gewonnenem Synthesegas vor, um Dampf zu erzeugen. Ein Vergasungsprozess und ein Gasfermentationsprozess können integriert werden, wobei Abgas aus dem Fermentationsprozess für die Flamme verwendet wird, um Teer und andere Verbindungen aus dem durch einen Vergasungsprozess erzeugten Synthesegas zu verbrennen. Die Integration kann dadurch erreicht werden, dass Teer und andere Verbindungen aus dem Industriegas oder dem aus der Vergasung stammenden Synthesegas mit Hilfe eines Adsorptionsmittels entfernt und das Adsorptionsmittel mit dem Abgas aus dem Gasfermentationsprozess regeneriert wird. Das mit dem desorbierten Teer und anderen Verbindungen angereicherte Endgas kann zur Dampferzeugung in einem Dampfkessel verwendet werden, und der Dampf kann für eine Vielzahl von Zwecken genutzt werden, einschließlich der Stromerzeugung, um z. B. einen Kompressor des Gasvergärungsprozesses anzutreiben.

Die Erfindung, die in WO2013/08539 5A1 offenbart wird, bezieht sich auf ein Verfahren zur Verringerung des Ammoniakverlustes und des Geruchs aus organischem Material oder Abfall in die Atmosphäre. Ein Plasmagenerator wird eingesetzt, um organische Abfälle und Gülle mit einer Mischung aus sauren Nitraten und Nitriten zu veredeln. Die Erfindung betrifft auch eine saure Nitratlösung, die zur Verringerung des Ammoniakverlustes und des Geruchs aus organischem Material oder Abfall in die Atmosphäre geeignet ist, sowie ein Verfahren zur Herstellung einer solchen sauren Nitratlösung. Die Erfindung umfasst ferner Anlagen zur Verringerung des Ammoniakverlustes und des Geruchs aus organischem Material oder Abfall in die Atmosphäre.

Ein Verfahren zur Herstellung von Biogas ist in der Druckschrift US 2014 0193 898 A1 vorgesehen. Das Verfahren umfasst die Schritte des Bereitstellens einer Polykultur von Wasserpflanzen in einem Wachstumssystem; des kontinuierlichen Bereitstellens von Wasser, Kohlendioxid, Luft und Nährstoffen für die in dem Wachstumssystem enthaltene Polykultur; des Wachstums der Polykultur für eine Zeit, die ausreicht, um eine auf Wasserpflanzen basierende Biomasse zu erzeugen; des Abziehens eines Teils der in dem Wachstumssystem enthaltenen auf Wasserpflanzen basierenden Biomasse; und des Behandelns der abgezogenen auf Wasserpflanzen basierenden Biomasse, um ein Biogas zu erzeugen.

In allen bekannten Systemen der o.g. Anlagen ist der durchschnittliche Abbaugrad der organischen Bestandteile 75-80%. Das heißt, dass ein Drittel bzw. ein Viertel im Verhältnis zu der abgebauten Organischen Substanz, nicht erschlossen wird .

Die Ursache dafür liegt in der Struktur, in welcher diese Bestandteile vorliegen. Im Falle von Gärresten sind es zumeist Lignozellulose-Strukturen, die eine natürlichen Abbau verhindern.

### Aufgabe der Erfindung

Die Aufgabe der Erfindung bestand darin, die o.g. Nachteile der bekannten technischen Lösungen zu beseitigen.

### Lösung der Aufgabe

Die Aufgabe wurde gemäß den Merkmalen der Patentansprüche gelöst. Durch das beschriebene erfindungsgemäße System ist es nun möglich, diese - bisher nur schwer aufschließbaren Strukturen weitestgehend aufzuschließen und so nutzbar zu machen.

Das bedeutet, dass ein Drittel bzw. ein Viertel mehr Gas (Methan) produziert wird.

Auf der anderen Seite bedeutet es die Verminderung von Nachvergärungspotential mit den damit verbundenen ökologischen Problemen sowie die Vermeidung bzw. Minimierung von aufwendigen Nachbehandlungsverfahren wie Kompostierung oder Schlammverbrennung.

Die Behandlungsstufen sind folgende:

### 1. Nasszerkleinerung

Auflösung grober Faserverbünde und Partikel, Verkleinerung der Partikel Größe und Vergrößerung der Oberfläche der Suspensionsbestandteile, vorzugsweise mittels Rotations-Schneidern. Dabei werden feste und flüssige Bestandteile separiert, wobei nur flüssige Fraktionen mit unterschiedlichem Eindickungsgrad austreten.

### 2. Druckbehandlung

Eine Separator-Stufe führt zu einer Druckdifferenz, welche zur weiteren Behandlung der Partikel der Suspension führt, so dass der Prozess aus der Nasszerkleinerung noch einmal deutlich verstärkt wird, Zerkleinerung der Partikel und Vergrößerung der Oberflächen werden noch einmal maximiert.

Die Druckdifferenz spielt sich zwischen -0,3 bar und +1bar, bezogen auf Atmosphärendruck, ab, mit einem Differenzdruck von 0,5 bar bis zu einigen bar in Abhängigkeit von dem Zuführsystem bzw. Zuführpumpe, wobei die Erreichung eines Unterdruckes unter atmosphärischen Druck erforderlich ist.

### 3. Mechanische Behandlung

Durch die drehenden Bauteile des Separators wird unmittelbar vor dem Durchtritt durch die Siebstruktur auch eine mechanische Zerkleinerung erzielt, die die vorherigen Schritte ergänzt.

### 4. Entgasung

Der Separator wird so betrieben, dass auf der Rückseite des Separationssiebes ein Unterdruck erzeugt wird. Der auf der Unterdruckseite des Separationssiebes eintretende zusätzliche Effekt ist die Entgasung des Substrates. Zell- und Substrataufschluss Vorgänge im Unterdruckbereich sind die Folge.

Durch den Unterdruck werden sehr komplexe Vorgänge ausgelöst.

Der an der Oberfläche der Siebstruktur auftretende Unterdruck bewirkt umgekehrt wirkende Scherkräfte ähnlich wie sie bei den Implosionen durch den Ultraschall hervorgerufen werden.

Bezogen auf eine noch intakt geschlossene Zellmembran eines Einzellers wird hier ähnlich ein Druckunterschied auf die Zellmembran ausgeübt, welcher vom Innenraum der Zelle zum die Membran umgebenden Druck ein ähnlich negatives Druck Verhältnis herstellt, wie es in der Nähe zu einem Implosionsvorgang (Kavitationsvorgang) erscheint.

Für beschädigte Zell-Membranen oder partikulare Verbünde wirkt dieses Druckverhältnis umso stärker.

Dass die Löslichkeit von Gasen in Flüssigkeiten von Druck und Temperatur abhängig sind, hat hier einen verstärkten Einfluss auf die Entgasung, da hier der Unterdruck in einer Behandlungsstufe abläuft, welcher durch die Behandlung im Verfahren zu einer deutlichen Temperaturerhöhung geführt hat. Mechanische Behandlungsstufen und Ultraschall und Plasma Behandlung führen in Addition zu deutlichen Temperaturerhöhungen.

### 5. Gasnutzung, Aufbereitung und Umwandlung zu Wertstoffen

Das durch die Entgasungsvorgänge im speziellen Separationsprozess austretende Gas hat ein vielfaches Volumen im Verhältnis zu dem zur Behandlungsrestaufbereitung bereitgestellten Substratmengen. Deshalb ist eine Nutzung über Aufbereitung und Umwandlung eine angemessene Maßnahme, um den Behandlungsprozess effizient und nachhaltig zu gestalten.

Für diese Behandlungsstufe wird eine Plasmaentladung über einen Stack Reaktor, eine Microwellenentladung oder eine Plasma Torch Behandlung vorgenommen, durch welche folgende Reaktionen erfolgen:

CO₂ + 4 H₂ > CH₄ + 2 H₂O (Gleichung 1)

CO₂ + 3 H₂ > CH₃OH + H₂O (Gleichung 2)

CO₂ + H₂ > CO + H₂O (Gleichung 3)

CO₂ + CH₄ > 2 CO + 2 H₂ (Gleichung 4)

Die aus dem Gas der Eingangsstufe des Verfahrens gewonnenen CO₂ Mengen werden durch gezielte Plasmabehandlung - gemäß den Gleichungen 1 bis 4 - zu den angegeben werthaltigen Gasfraktionen umgewandelt. Sie können dem Anaerob-Prozess der Ausgangsstoffe zugegeben oder für andere externe Wertschöpfungskreise genutzt werden.

### 6. Beschallung mit Hochleistungsultraschall

Durch die Ultraschallbehandlung im nachfolgenden Schritt wird hier noch einmal eine Verdopplung der Aufschluss-Effekte durch Kavitation im Verhältnis zu nicht vorbehandeltem entgastem Substrat erzielt.

Die Zunahme der durch den Ultraschall in lösliche CSB Anteile aufgeschlossene organische Substanz des Gärrestes ist ein Maß für die enorme Steigerung der Nutzung bisher ungenutzter Organik aus dem Gärrest. Diese kann dem Biogasprozess wieder zugeführt werden und die Effizienz der Biogasproduktion der betreffenden Anlage deutlich erhöhen. CSB ist der chemische Sauerstoff-Bedarf, hier gibt es den gesamt CSB, welcher ein Maßstab für den organischen Anteil in einer Suspension ist, der sich in Gesamt-CSB und gelösten CSB aufteilt. Je höher der gelöste CSB nach der Behandlung, desto effektiver ist das Behandlungsverfahren. Gelöster CSB ist für Mikroorganismen direkt verfügbar bzw. steht für chemische Prozesse zur Verfügung.

Der erhöhte partikuläre Aufschluss in dieser Stufe hat eine große Bedeutung für die Effizienz für nachgeschaltete Gärrestaufbereitungsverfahren zur Herstellung von Düngerfraktionen und Kondensationsvorgängen zur Wasserabtrennung aus dem Behandlungsreststoff.

### 7. Beschallung mit Hochleistungsultraschall und Mikrowellen Plasma

Der aus der mit erhöhtem Druck behandelte Substrat aus der Separationsbehandlung wird dem neuartigen, dem Separationsteil angeschlossenen Bauteil mit einer UltraschallQuelle und der Zündung eines Microwellen-Plasmas in dem vom behandelten Separat durchströmten Behandlungsbauteil mit Gasabscheidung geleitet. Durch diese Kombination wird noch ein zusätzlicher Effekt der s.g. Desintegration ausgelöst, um eine weitere Steigerung der Auflösung von bisher nicht gelöster Organik aus dem Gärrest zu erzielen.

### 8. Abspaltung von Wasserstoff

Durch die gemeinsame Anwendung von Ultraschall und Microwellenplasma ist es gelungen, bisher nicht am Biogasbildungsprozess teilhabende Substanzen aus dem Gärsubstrat nutzbar zu machen.

Diese sind zum Beispiel Ammoniumverbindungen und Ammoniak, aus denen Wasserstoff freigesetzt wird. Mit diesem Wasserstoff ist es möglich, bei der Rückführung in den Fermentationsraum der Biogasanlage den Methangehalt des Biogases zu steigern.

Bei Zugabe von Ammoniumsulfat oder anderen durch Wasserstoffverbindungen gekennzeichnete Substanzen ist es möglich, zusätzliche Wasserstoff Mengen zu erzeugen.

Das behandelte Substrat aus der Druckseite der Separationsstufe sowie das überschüssige Unterdruck behandelte, nicht direkt einem Verfahren zur Behandlungsrest aufbereitendem Verfahren zugeführte Substrat, wird über die Ultraschall-Plasma Behandlung geleitet, sowie dann anschließend dem anaeroben Fermentationssystem, aus dem das in dem neuen beschriebenen Verfahren zu behandelnde Substrat entnommen wurde, zugeführt.

Das wie beschrieben behandelte Substrat nimmt den Weg zurück in den Fermenter und wird als zusätzliche organische Ressource, Kohlenstoffquelle den aeroben und anaeroben Abwasser- und Abfallaufbereitungsanlagen sowie Biogaserzeugungsanlagen wieder zugeführt.

Durch die Ultraschall-Plasmabehandlung der in den vorbeschriebenen Verfahrensstufen vorbehandelten und entgasten Suspension wird die Wirkung des Ultraschalls und dessen Kombination mit dem in der Flüssigkeit gezündeten Plasma effizienter und stärker. Es wird ein erhöhter Aufschlussgrad in Richtung gelösten organischen Verbindungen erzielt und es wird sehr effektiv Wasserstoff abgespalten.

Durch die wiederholte Rezirkulation in den Fermenter und neue intensive mehrstufige Behandlung gelingt es, die Abbauprozesse der organischen Inhaltsstoffe wesentlich zu beschleunigen, eine nachhaltige und umfassende Ausnutzung der organischen Ressourcen aus den aeroben und anaeroben Abwasser- und Abfallaufbereitungsanlagen sowie Biogaserzeugungsanlagen zu erzielen.

Durch diese das Behandlungsverfahren charakterisierenden Behandlungsstufen ist es möglich, eine neue Qualität der Ausnutzung und des Abbaus von flüssigen organischen Suspensionen zu erzielen. Der Abbaugrad der Organik und die damit verbundene Umweltentlastung und Emission-Vermeidung aus Abfällen, Abwasserschlämmen, Wirtschaftsdüngern erzeugt eine Recycling-Möglichkeit für organische Abfälle und Wirtschaftsdünger in einem Kreislaufverfahren ohne nicht-nutzbare Reststoffe.

Die in dem Verfahren enthaltene Abspaltung von Wasserstoff und andern werthaltigen Gasen aus den zu behandelnden Suspensionen und Flüssigkeiten stellt einen neuen Weg zur Nutzung von Ressourcen bereit - nicht nur aus organischen Substraten, sondern auch aus anorganischen und zum Teil toxischen Verbindungen aus den aufzubereitenden Stoffen.

Das erfindungsgemäße Verfahren ist auch kombinierbar mit einem Vakuum Verdampfungs-Verfahren. Durch diese Kombination wird unter Einsatz von Abwärme bis zu 75% des Wassers aus Gärresten verdampft und als Kondensat abgeführt oder genutzt.

Dieses Wasser enthält dann keine relevanten Inhaltsstoffe und ist ökologisch vollkommen neutral. Auf der anderen Seite entstehen zwei flüssige Fraktionen welche als Dünger dienen, da sie alle in den Gärresten enthaltenen Dünger relevanten Inhaltsstoffe aufkonzentrieren, ein sogenannter Dickschlamm und eine Ammoniumsulfat Lösung.

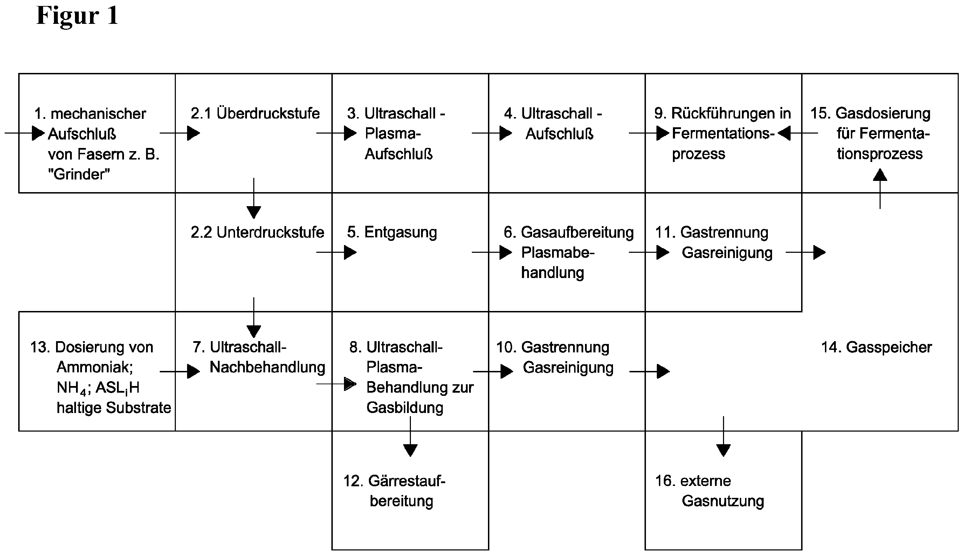

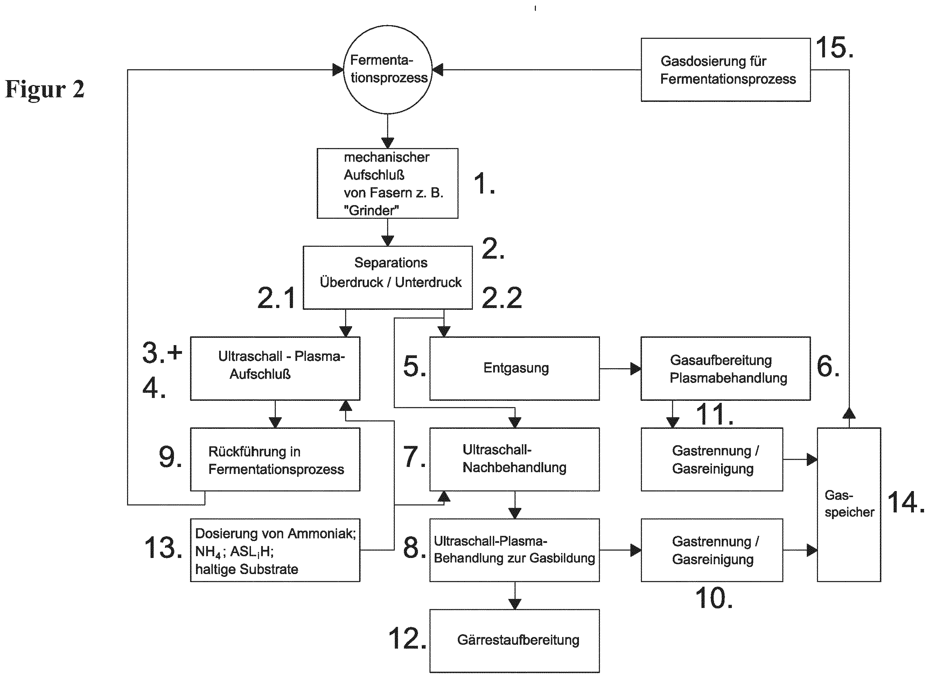

Das erfindungsgemäße Verfahren wird anhand der Figuren 1 und 2 näher erläutert.

Figur 1 (Schema 1) zeigt den groben Verfahrensablauf, während Figur 2 (Schema 2) die Einordnung des Verfahren-Schemas in eine Anwendung zwischen einem FermentationsSystem(Biogasanlage) und einer Gärrestbehandlungsanlage darstellt.

Figur 3 zeigt eine neu entwickelte Reaktionskammer für kombinierte zeitgleiche Plasma und Ultraschallbehandlung. Diese zeichnet sich durch besonders kompaktes Gehäuse aus, welches die räumlichen Anforderungen für das komplette Gerät um ca. ein Drittel reduziert.

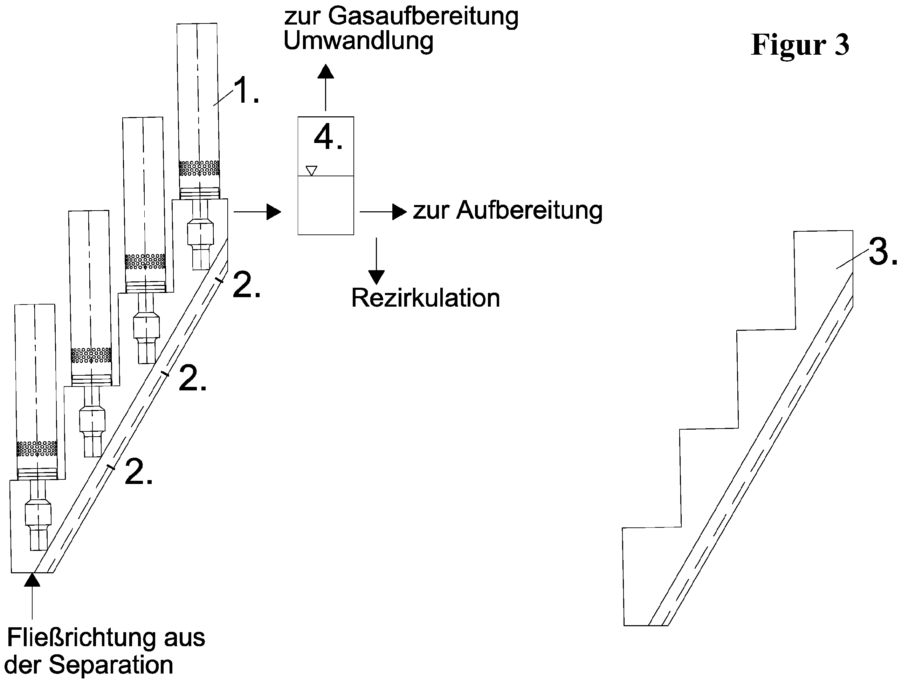

Die Erfindung umfasst auch eine Reaktorkammer für die kombinierte gleichzeitige Suspensionsbehandlung. Gemäß Figur 3 wird diese Kammer aufstrebend durchströmt, um erstens eine reine Ultraschallbehandlung über die integrierten Sonotroden durchzuführen und zweitens eine parallele kombinierte zeitgleiche Ultraschall- und Plasma-Behandlung durchzuführen. An den Elektroden werden spezielle Koppler-Mikrowellen-Plasmen gezündet, bzw. diese durch Torch Plasmen erzeugende Bauteile ersetzt

In diesem Reaktor gibt es Zero-Schlupf sowie ideale Gas- und Sediment-Abfuhr auf Gravitationsbasis.

Die zu behandelnde Substratmenge befindet sich in der gesamten Behandlungszeit in der Kavitationszone der Sonotroden. Die Anströmung bzw. Durchströmung wird durch mechanische plus thermodynamische Energie erzielt.

Alle von Neis/Nickel aufgestellten Regeln wurden eingehalten bzw. berücksichtigt. Der Wirkungsgrad in diesem Reaktor übersteigt die bisherigen Ergebnisse sehr deutlich.

### Erläuterung zu den Figuren:

Der grobe Verfahrensablauf (Figur 1) umfasst folgende 16 Schritte:

| | | | |
|---|---|---|---|
| 1. | mechanischer Aufschluss von Fasern, z.B. "Grinder" | 8. | Ultraschall-Plasma-Behandlung zur Gasbildung |
| 2. | Separationsstufen | 9. | Rückführung in Fermentationsprozess |
| 2.1. | Überdruckstufe | 10. | Gastrennung / Gasreinigung |
| 2.2. | Unterdruckstufe | 11. | Gastrennung / Gasreinigung |
| 3. | Ultraschall-Plasma-Aufschluss | 12. | Gärrestaufbereitung |
| 4. | Ultraschall-Aufschluss | 13. | Dosierung von Ammoniak, NH4; ASL, H; haltige Substrate |
| 5. | Entgasung | 14. | Gasspeicher |
| 6. | Gasaufbereitung Plasmabehandlung | 15. | Gasdosierung für Fermentationsprozess |
| 7. | Ultraschall-Nachbehandlung | 16. | externe Gasnutzung |

In Figur 2 ist das Übersichtsschema 2 abgebildet.

### Bezugszeichenliste für Figur 3

- 1: Ultraschall-Sonotroden
- 2: Microwellen - Plasma - Elektrode Coupler
- 3: Korpus zur Aufnahme von 1. und 2.
- 4: Gasabscheider

## Patentansprüche

1. Verfahren zum mehrstufigen Aufschluss von organischen Substraten inhomogener Suspensionen zur Bildung von Methan und Wasserstoff, **gekennzeichnet durch** folgende Schritte:
a) Mechanische Zerkleinerung
b) Separationsstufe durch Behandlung mit Unterdruck und Überdruck
c) Ultraschall Behandlung oder/und Kombinierte Ultraschall-Plasma-behandlung
d) Ultraschallbehandlung eines zweiten Stromes aus der Stufe b),
wobei diese Behandlungsstufen hintereinander geschaltet sind.

2. Verfahren nach Anspruch 1, **dadurch gekennzeichnet, dass** bei der Behandlung mittels Unterdruck eine Entgasung dieses behandelten Teils der Suspension erfolgt.

3. Verfahren nach Anspruch 2, **dadurch gekennzeichnet, dass** die freigesetzten Gase einer Plasmabehandlung unterzogen werden, wobei CO₂ zu Methan, CO, Methanol mittels H₂ umgewandelt wird.

4. Verfahren nach Anspruch 1 und 2, **dadurch gekennzeichnet, dass** die entgasten flüssigen Rückstände mittels Ultraschall oder/ und einer Kombination aus Ultraschall und Plasma nachbehandelt werden, wobei weitere Gase freigesetzt werden.

5. Verfahren nach Anspruch 4, **dadurch gekennzeichnet, dass** den entgasten Rückständen Ammoniak oder Ammoniumverbindungen bzw. andere flüssige oder feste Wasserstoff enthaltende Stoffe zugegeben werden, die - infolge der Ultraschall und Plasmabehandlung - Wasserstoff abgeben.

6. Verfahren nach einem der Ansprüche 1 bis 5, **dadurch gekennzeichnet, dass** die Plasmabehandlung der Gasfraktion mittels Plasmaentladung, vorzugsweise über einen Stack Reaktor, eine Microwellenentladung oder eine Plasma Torch Behandlung erfolgt.

7. Verfahren nach einem der Ansprüche 1 bis 6, **dadurch gekennzeichnet, dass** die mechanische Zerkleinerung mittels Rotations-Schneidern erfolgt.

8. Vorrichtung zur Durchführung des Verfahrens gemäß einem der Ansprüche 1 bis 7, umfassend:
a) Rotations-Schneider
b) Separationssystem aus dem nur flüssige Fraktionen mit unterschiedlichem Eindickungsgrad austreten
c) Geräte zur Druck- bzw. Unterdruckerzeugung
d) Geräte zur Plasmabehandlung
e) Geräte zur Ultraschallbehandlung
f) Geräte zur kombinierten Plasma und Ultraschallbehandlung in gemeinsamen Reaktionskammern
g) Gasspeicher.

9. Reaktorkammer für kombinierte gleichzeitige Suspensionsbehandlung zur Durchführung einer Ultraschallbehandlung und einer kombinierten zeitgleichen Ultraschall und Plasma Behandlung gemäß einem der Ansprüche 1 bis 7, umfassend Ultraschall-Sonotroden einerseits und Koppler-Mikrowellen-Plasmen oder Torch-Plasmen erzeugende Bauteile anderseits, **dadurch gekennzeichnet, dass** die Ultraschall- und Plasma erzeugenden Geräte nacheinander angeordnet sind, so dass diese von der Suspension nacheinander durchströmt werden.

10. Kombination des Verfahrens gemäß einem der Ansprüche 1-6 oder der Vorrichtung gemäß Anspruch 8 oder 9 mit einem Vakuum Verdampfungs-Verfahren.
